# EUROPEAN PATENT SPECIFICATION

(11) **EP 2 735 263 B1**
(45) Date of publication and mention of the grant of the patent: **24.02.2021**
(21) Application number: 12815220.4
(22) Date of filing: 20.06.2012
(51) Int. Cl.: A61B 3/10, A61B 3/00, G07C 9/00, A61B 3/11, G06F 21/32, G06K 9/00

(54) **MOBILE DEVICE AND PUPIL RECOGNITION METHOD THEREFOR**
MOBILE VORRICHTUNG UND PUPILLENERKENNUNGSVERFAHREN DAFÜR
DISPOSITIF MOBILE ET PROCÉDÉ DE RECONNAISSANCE DE PUPILLE POUR CELUI-CI

(30) Priority: 18.07.2011 CN 201110200966
(43) Date of publication of application: 28.05.2014
(73) Proprietor: Huizhou TCL Mobile Communication Co., Ltd., Huizhou, Guangdong 516006 (CN)
(72) Inventor: ZHANG, Fan, Huizhou Guangdong 516006 (CN)
(74) Representative: Brachmann, Roland W.
(86) International application number: PCT/CN2012/077192
(87) International publication number: WO 2013/010421

(56) References cited:
- WO-A1-03/090174
- WO-A2-2007/094988
- CN-A- 1 692 374
- CN-A- 1 805 701
- CN-A- 102 354 359
- KR-A- 20040 110 284
- US-A1- 2005 031 173
- US-B1- 6 267 477
- US-B1- 6 314 401
- US-B2- 7 805 372
- PU, ZHAOBANG ET AL.: 'Development and Application of Iris Recognition Technology' OPTICS AND PRECISION ENGINEERING vol. 12, no. 3, June 2004, pages 316 - 322, XP008173250
- PU ZHAOBANG ET AL: "Development and Application of Iris Recognition Technology", GUANGXUE JINGMI GONGCHENG - OPTICS AND PRECISION ENGINEE, ZHONGGUO KEXUEYUAN CHANGCHUN JINGMI JIXIE YANJIUSUO, CHANGCHUN, CN, vol. 12, no. 3, 1 June 2004 (2004-06-01), pages 316-322, XP008173250, ISSN: 1004-924X

## Description

The present invention relates to a mobile device, in particular relates to a mobile device using the pupil characteristic information of a user to perform identity verification.

An existing mobile device, such as a mobile phone, will have its all functions and files displayed on the screen after the startup. As for the file or program which can only be accessed with a corresponding authorization, a password will be set for it traditionally. However this kind of encryption has a relative lower security level.

The patent "Method for Realizing Related Mobile Communication Terminal Function by Fingerprint Recognition" (Application No.: 200910200922.6) discloses a security protection method using fingerprint recognition. Although the fingerprint recognition has the property of uniqueness too, it can still be risky because a fingerprint is easy to be stolen due to the touch of the finger to too many things in daily life.

WO 2007/094988 A2 discloses a method and system for controlling a vehicle given to a third party. The system includes a system controller; a mode-indicating device coupled to the system controller; and an authenticator coupled to the system controller. The system controller is adapted to communicate a driving restriction to the vehicle upon an activation of the mode-indicating device by an authorized driver and until a deactivation of the mode-indicating device by the authorized driver, the system controller is adapted to restrict the activation and the deactivation of the mode-indicating device unless the authorized driver has been authenticated by the authenticator. The authenticator may include an iris authenticator.

Pu Zhao-bang, Yang Fan, Chen Bing-yi, Chen Shi-zhe: "Development and application of Iris recognition technology"; Optics and Precision Engineering, Vol.12, No.3, June 2004, discloses an automatic cash dispenser with iris recognition function to replace a password entry. Further disclosed is the use of iris recognition in the fields of finance, public security, personnel management, medical care, electronic trade, intelligent access control systems, and channel control.

KR 20040110284 A discloses a locking control method for a mobile phone using iris recognition to prevent a person from using the mobile phone without permission of the owner of the mobile phone by judging whether the person is permitted in the use of the mobile phone through the iris recognition. To this effect, the iris of a user is photographed, and the photographed image signal is stored in a memory. The iris of a certain user requesting iris recognition is photographed, and the photographed image signal is compared with the stored image signal. If an identical iris image signal exists, the use of a mobile phone is permitted. If the identical iris image signal does not exist, it is impossible to use all services except for a specific service.

A technical solution employed in an embodiment of the present invention to solve the above mentioned technical issue is: providing a mobile device, which comprises a pupil information collecting module for collecting pupil characteristic information of a user and a master control module; and wherein
the mobile device is a mobile phone or a PDA;
the pupil information collecting module comprises:
- a pupil scanner for collecting pupil characteristic information of a user;
- a driver for driving the pupil scanner to collect the pupil characteristic information of the user;
- a microprocessor for controlling the pupil scanner and the driver, calculating the pupil characteristic information and sending it to the master control module;
a master control module for receiving the pupil characteristic information of the user when the user accesses a controlled unit, and for determining, on the basis of the pupil characteristic information of the user, if the user is allowed to access to the controlled unit;
wherein, the controlled unit comprises system programs, applications or files of the mobile device, the pupil characteristic information comprises the distance between the pupil and the pupil scanner, the pupil luminance, the curvature of the pupil lens, and the lines of the pupil capillaries.

According to a preferred embodiment of the present invention, the pupil scanner comprises:
- an ultrasonic transmitting and receiving unit for acquiring the distance between the pupil and the pupil scanner;
- a photosensitive diode for collecting the pupil luminance;
- a laser emission unit for providing a illumination source to facilitate the photosensitive diode to collect the pupil luminance;
- a signal processing unit for optimizing the signals collected by the photosensitive diode;
- an interface unit for connecting the ultrasonic transmitting / receiving unit, the photosensitive diode and the laser emission unit to the microprocessor.

According to a preferred embodiment of the present invention, the signal processing unit comprises at least an amplifier and a filter.

Another technical solution employed in the present invention in order to solve the above mentioned technical issue is: providing a pupil recognition method for a mobile device as described above, which comprises the steps of:
- setting pupil recognition for a controlled unit of the mobile device;
- collecting pupil characteristic information of a user able to access the controlled unit by using the pupil information collecting module of the mobile device;
- setting an access authorization for the pupil characteristic information of the user by using the master control module of the mobile device.

According to a preferred embodiment of the present invention, when using the pupil information collecting module to collect the pupil characteristic information of the user able to access the controlled unit, if the collected pupil characteristic information is found to be unmatched with the requirement, then the user is prompted to perform collection again until the pupil characteristic information matching with the requirement is collected, and the pupil characteristic information matching with the requirement is stored to the internal memory.

According to a preferred embodiment of the present invention, the user accessing the controlled unit of the mobile device comprises following steps:
- prompting the user to perform identity verification;
- collecting the pupil characteristic information of the user by the pupil information collecting module;
- comparing the collected pupil characteristic information of the user with the pupil characteristic information stored in the internal memory by the master control module to determine if the pupil characteristic information is the one that is allowed to access the controlled unit, and if yes, then granting the access, otherwise denying the access.

According to a preferred embodiment of the present invention, when the pupil information collecting module collects the pupil characteristic information of the user, it comprises following steps:
- resetting the pupil information collecting module;
- driving the pupil scanner by the driver to collect the pupil characteristic information of the user;
- judging by the microprocessor if the pupil characteristic information collecting is finished, if yes, calculating the pupil characteristic information collected by the pupil scanner and sending it to the master control module, and if not, then continuously driving the pupil scanner to perform collection.

To sum up, the mobile device and the pupil recognition method thereof disclosed in the present invention perform identity verification through the pupil characteristic information of the user, providing great security.

Described above is the summary of the technical solutions of the present invention, which can be implemented according to the content of the description in order to understand the technical means of the present invention more clearly, and the present invention will be further described with reference to the preferred embodiments and the accompanied drawings for a better understanding of the above mentioned and other purposes, characters and advantages of the present invention.

The aforementioned characteristics, features and advantages of the invention as well as the way they are achieved will be further illustrated in connection with the following examples and considerations as discussed in view of the figures.
- Figure 1: is a schematic diagram showing the module structure of a mobile device according to a preferred embodiment of the present invention;
- Figure 2: is a schematic diagram showing the module structure of a mobile device according to another preferred embodiment of the present invention;
- Figure 3: is a schematic diagram showing the module structure of the pupil information collecting module shown in Figure 1;
- Figure 4: is a schematic diagram showing the module structure of the pupil scanner shown in Figure 3;
- Figure 5: is a flow diagram of setting a pupil recognition method for a mobile device according to a preferred embodiment of the present invention;
- Figure 6: is a flow diagram showing the user accessing a controlled unit in the mobile device;
- Figure 7: is a work flow diagram showing the pupil information collecting module collecting the pupil characteristic information.

The present invention will be further described in detail with the accompanied drawings and embodiments.

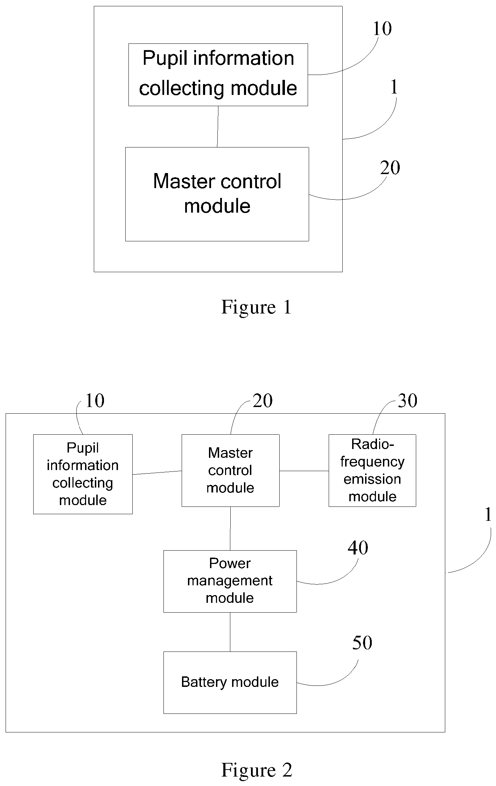

Referring to Figure 1, the present invention provides a mobile device 1, such as a mobile phone or a PDA. The mobile device 1 comprises a pupil information collecting module 10 and a master control module 20, wherein, the pupil information collecting module 10 is used to collect the pupil characteristic information of a user; the master control module 20 receives the pupil characteristic information of the user when the user accesses a controlled unit, and determines, on the basis of the pupil characteristic information of the user, if the user is allowed to access to the controlled unit. In the embodiment of the present invention, the controlled unit comprises system programs, applications or files of the mobile device 1.

Referring to Figure 2, in another embodiment of the present invention, the mobile device 1 comprises a pupil information collecting module 10, a master control module 20, a radio-frequency emission module 30, a power management module 40 and a battery module 50, wherein, the radio-frequency emission module 30 is used to communicate with an external network, the power management module 40 is used to divide the voltage of the battery module 50 to each function module, and the battery module 50 is used to provide the power for the system to work.

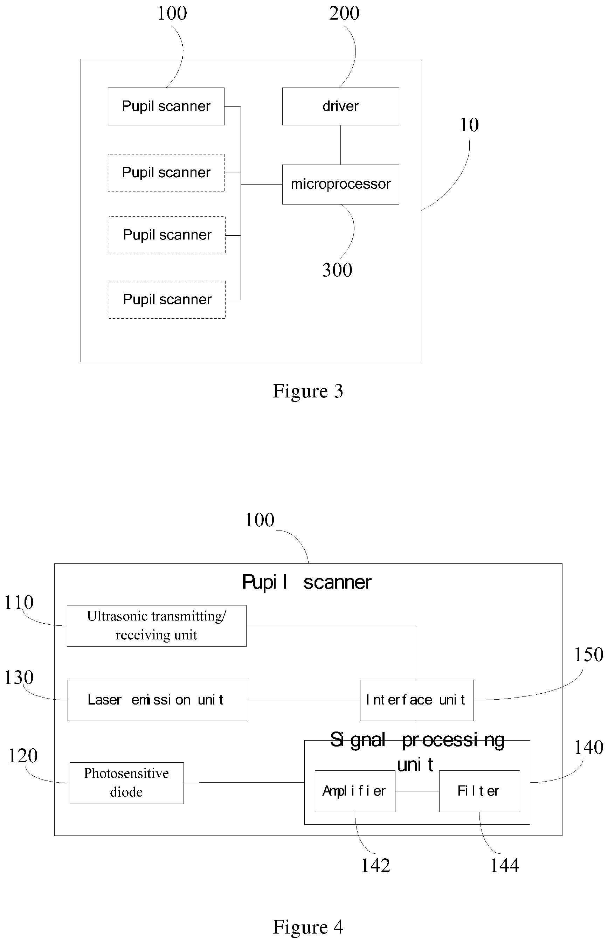

Referring to Figure 3, in the embodiment of the present invention, the pupil information collecting module 10 mainly comprises a pupil scanner 100, a driver 200 and a microprocessor 300.

Wherein, the pupil scanner 100 is used to collect the pupil characteristic information of a user; responding to the order of the microprocessor 300, the driver 200 is used to drive the array of the pupil scanner 100 to multiple positions to collect the pupil characteristic information in the corresponding positions, the driver 200 can be a motor, etc.; the microprocessor 300 is used to control the pupil scanner 100 and the driver 200, calculate the pupil characteristic information and send it to the master control module 20.

In the embodiment of the present invention, the pupil characteristic information comprises the distance between the pupil and the pupil scanner 100, the pupil luminance, the curvature of the pupil lens, and the lines of the pupil capillaries.

To be specific, referring to Figure 4, in the embodiment of the present invention, the pupil scanner 100 further comprises a ultrasonic transmitting / receiving unit 110, a photosensitive diode 120, a laser emission unit 130, a signal processing unit 140 and an interface unit 150.

Wherein, the ultrasonic transmitting / receiving unit 110 is used for acquiring the distance between the pupil and the pupil scanner 100; the photosensitive diode 120 is used for collecting the luminance of the pupil; the laser emission unit 130 is used for providing a illumination source to facilitate the photosensitive diode 120 to collect the pupil luminance; the signal processing unit 140 is used for optimizing the signals collected by the photosensitive diode 120, in the embodiment, the signal processing unit 140 comprises at least an amplifier 142 for amplifying the weak signals of the photosensitive diode 120 and a filter 144 for performing band-pass filtering on the collected and amplified noises; the interface unit 150 is used for connecting the ultrasonic transmitting / receiving unit 110, the photosensitive diode 120 and the laser emission unit 130 to the microprocessor 300.

And next, the pupil recognition method of the mobile device 1 provided by the embodiment of the present invention will be introduced with reference to above mentioned hardware descriptions.

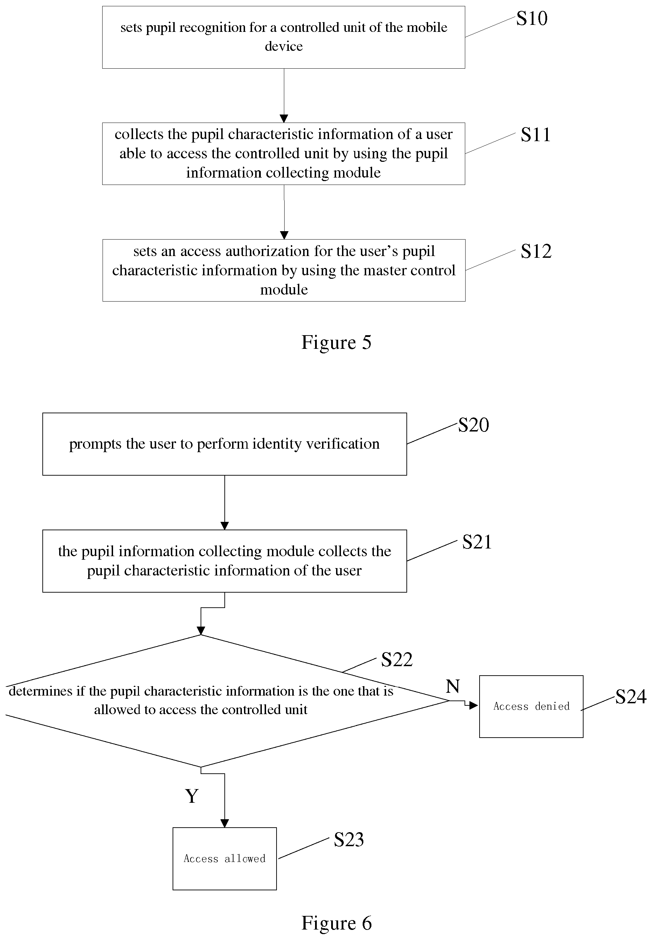

Referring to Figure 5, the Figure 5 shows the flow diagram for setting the pupil recognition method, which comprises following steps:
S10: setting pupil recognition for a controlled unit of the mobile device 1, for example setting the pupil recognition to a system program (startup program) of the mobile device 1, or to a dedicated application or an important file of the mobile device 1;
S11: collecting the pupil characteristic information of a user able to access the controlled unit by using the pupil information collecting module 10 of the mobile device 1;
S12: setting an access authorization for the pupil characteristic information of the user by using the master control module 20 of the mobile device 1, the setting type depends on the specific application circumstance, for example, it can be set that an ordinary user has a read only permission, while a special user has modification permission.

It is worth mentioning that in order to collect the pupil characteristic information of the user accurately, when using the pupil information collecting module 10 to collect the pupil characteristic information of the user able to access the controlled unit, if the collected pupil characteristic information is found to be unmatched with the requirement, then the user is prompted to perform collection again until the pupil characteristic information matching with the requirement is collected, and then the pupil characteristic information matching with the requirement is stored into the internal memory.

Next referring to Figure 6, the Figure 6 shows the flow diagram of a user accessing a controlled unit in the mobile device 1, which comprises following steps:
S20: prompting the user to perform identity verification, for instance popping up a verification interface on the display screen (not shown) of the mobile device 1;
S21: the pupil information collecting module 10 collecting the pupil characteristic information of the user;
S22: the master control module 20 comparing the collected pupil characteristic information of the user with the pupil characteristic information stored in the internal memory to determine if the pupil characteristic information is the one that is allowed to access the controlled unit;
S23: if yes, then granting the access;
S24: if not, then denying the access.

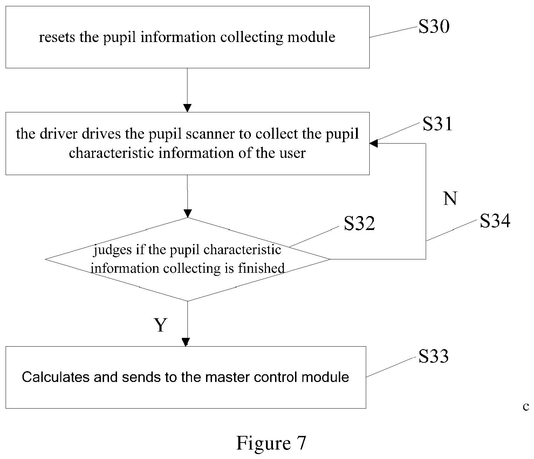

Referring to Figure 7, Figure 7 is a work flow diagram of the pupil information collecting module 10 collecting the pupil characteristic information of the user, which comprises following steps:
S30: resetting the pupil information collecting module 10, that is the driver 200 drives the pupil scanner 100 to the initial position;
S31: the driver 200 driving the array of the pupil scanner 100 to multiple positions to collect the pupil characteristic information of the user in corresponding positions, that is acquiring the distance between the pupil and the pupil scanner 100 by the ultrasonic transmitting / receiving unit 110, collecting the luminance of the pupil by the photosensitive diode 120;
S32: the microprocessor 300 judging if the pupil characteristic information collecting is finished, by referring to if the driver 200 reaches the final position, if the driver 200 does not reach the final position, then the microprocessor 300 making the driver 200 continuously drive the pupil scanner 100 to collect the pupil characteristic information of the user;
S33 : if the pupil characteristic information collecting has been finished, that is the driver 200 has reached the final position, then calculating the pupil characteristic information collected by the pupil scanner 100 and sending it to the master control module, for example, calculating the three dimension information such as the curvature of the pupil lens, the capillary lines of the pupil and the cornea thickness through the parameters such as the distance between the pupil and the pupil scanner 100 and the luminance of the pupil;
S34: if not finished, then continuing step S31.

In conclusion, it is easy for those skilled in the field to understand that the mobile device and the pupil recognition therefor use the pupil characteristic information of a user to perform the identity verification, as the pupil characteristic information of the user is unique, cannot be stolen by others, thus providing extremely great security.

## Claims

1. A mobile device (1), comprising:
a pupil information collecting module (10) for collecting pupil characteristic information of a user;
a master control module (20) for receiving the pupil characteristic information of the user when the user accesses a controlled unit of the mobile device (1), and determines, on the basis of the pupil characteristic information of the user, if the user is allowed to access to the controlled unit;
and wherein
the mobile device (1) is a mobile phone or a PDA;
the controlled unit comprises system programs, applications or files of the mobile device (1)
the pupil information collecting module (10) comprises:
- a pupil scanner (100) for collecting the pupil characteristic information of the user;
- a driver (200) for driving the pupil scanner to collect the pupil characteristic information of the user;
- a microprocessor (300) for controlling the pupil scanner and the driver, calculating the pupil characteristic information and sending it to the master control module;
the pupil characteristic information comprises the distance between the pupil and the pupil scanner, the pupil luminance, curvature of the pupil lens, and lines of the pupil capillaries.

2. The mobile device (1) according to claim 1, wherein the pupil scanner (100) comprises:
- a ultrasonic transmitting / receiving unit (110) for obtaining the distance between the pupil and the pupil scanner (100);
- a photosensitive diode (120) for collecting the pupil luminance;
- a laser emission unit (130) for providing a illumination source to facilitate the collecting of the pupil luminance by the photosensitive diode (120);
- a signal processing unit (140) for optimizing the signals collected by the photosensitive diode (120);
- an interface unit (150) for connecting the ultrasonic transmitting / receiving unit, the photosensitive diode (120) and the laser emission unit (130) to the microprocessor (300).

3. The mobile device (1) according to claim 2, wherein the signal processing unit (140) comprises at least an amplifier (142) and a filter (144).

4. A pupil recognition method for a mobile device (1) according to any of the preceding claims, comprising following steps:
- setting pupil recognition (S10) for the controlled unit of the mobile device (1);
- collecting the pupil characteristic information (S11) of a user able to access the controlled unit by using the pupil information collecting module (10) of the mobile device (1);
- setting an access authorization (S12) for the pupil characteristic information of the user by using the master control module (20) of the mobile device (1), the pupil characteristic information comprising the distance between the pupil and the pupil scanner, the pupil luminance, curvature of the pupil lens, and lines of the pupil capillaries.

5. The pupil recognition method for a mobile device according to claim 4, wherein, when using the pupil information collecting module (10) to collect the pupil characteristic information of the user (S21) able to access the controlled unit, if the collected pupil characteristic information is found to be unmatched with the requirement (S32), then the user is prompted (S20) to perform collection again (S34) until the pupil characteristic information matching with the requirement is collected, and the pupil characteristic information matching with the requirement is stored into the internal memory.

6. The pupil recognition method for a mobile device according to any of claims 4 or 5, wherein, when the user accesses the controlled unit of the mobile device (1), it comprises following steps:
- prompting the user to perform identity verification (S20);
- collecting pupil characteristic information of the user by the pupil information collecting module (S21);
- comparing the collected pupil characteristic information of the user with the pupil characteristic information stored in the internal memory by the master control module to determine if the pupil characteristic information is the one that is allowed to access the controlled unit (S22), if yes, then granting the access (S23), otherwise denying the access (S24).

7. The pupil recognition method for a mobile device according to any of claims 4 to 6, wherein, when the pupil information collecting module (10) collects the pupil characteristic information of the user, it comprises following steps:
- resetting the pupil information collecting module (S30);
- driving the pupil scanner by the driver to collect the pupil characteristic information of the user (S31);
- judging by the microprocessor if the pupil characteristic information collecting is finished (S32), if yes, calculating the pupil characteristic information collected by the pupil scanner and sending it to the master control module (S33), if not, then continuously driving the pupil scanner to perform collection (S34).

## Patentansprüche

1. Mobile Vorrichtung (1), umfassend:
ein Pupilleninformations-Erfassungsmodul (10) zum Erfassen von charakteristischer Pupilleninformation eines Benutzers;
ein Master-Kontrollmodul (20), um die charakteristische Pupilleninformation des Benutzers zu empfangen, wenn der Benutzer auf eine zugangsbeschränkte Einheit der mobilen Vorrichtung (1) zugreift, und basierend auf der charakteristisch Pupilleninformationen des Benutzers zu bestimmen, ob dem Benutzer der Zugriff auf die zugangsbeschränkte Einheit gestattet ist;
und wobei
die mobile Vorrichtung (1) ein Mobiltelefon oder ein PDA ist;
die zugangsbeschränkte Einheit Systemprogramme, Anwendungen oder Dateien der mobilen Vorrichtung (1) umfasst;
das Pupilleninformations-Erfassungsmodul (10) umfasst:
- einen Pupillenscanner (100) zum Erfassen der charakteristischen Pupilleninformation des Benutzers;
- einen Treiber (200) zum Ansteuern des Pupillenscanners, um die charakteristische Pupilleninformation des Benutzers zu erfassen;
- einen Mikroprozessor (300) zum Steuern des Pupillenscanners und des Treibers, Berechnen der charakteristische Pupilleninformation und Senden derselben an das Master-Kontrollmodul;
wobei die charakteristische Pupilleninformation den Abstand zwischen der Pupille und dem Pupillenscanner, die Pupillenluminanz, die Krümmung der Pupillenlinse und Linien der Pupillenkapillaren umfasst.

2. Mobile Vorrichtung (1) gemäß Anspruch 1, wobei der Pupillenscanner (100) umfasst:
- eine Ultraschall-Sende/Empfangseinheit (110) zum Ermitteln des Abstands zwischen der Pupille und dem Pupillenscanner (100);
- eine lichtempfindliche Diode (120) zum Erfassen der Pupillenluminanz;
- eine Laseremissionseinheit (130) zum Bereitstellen einer Beleuchtungsquelle, um die Erfassung der Pupillenluminanz durch die lichtempfindliche Diode (120) zu erleichtern;
- eine Signalverarbeitungseinheit (140) zum Optimieren der von der lichtempfindlichen Diode (120) erfassten Signale;
- eine Schnittstelleneinheit (150) zum Verbinden der Ultraschall-Sende/Empfangseinheit, der lichtempfindlichen Diode (120) und der Laseremissionseinheit (130) mit dem Mikroprozessor (300) .

3. Mobile Vorrichtung (1) gemäß Anspruch 2, wobei die Signalverarbeitungseinheit (140) wenigstens einen Verstärker (142) und ein Filter (144) umfasst.

4. Pupillenerkennungsverfahren für eine mobile Vorrichtung (1) gemäß einem der vorstehenden Ansprüche, die folgenden Schritte umfassend:
- Einstellen der Pupillenerkennung (S10) für die zugangsbeschränkte Einheit der mobilen Vorrichtung (1);
- Erfassen der charakteristischen Pupilleninformation (Sll) eines Benutzers, der auf die zugangsbeschränkte Einheit zugreifen kann, unter Verwendung des Pupilleninformations-Erfassungsmoduls (10) der mobilen Vorrichtung (1);
- Einstellen einer Zugriffsberechtigung (S12) für die charakteristische Pupilleninformation des Benutzers unter Verwendung des Master-Kontrollmoduls (20) der mobilen Vorrichtung (1),
wobei die charakteristische Pupilleninformation den Abstand zwischen der Pupille und dem Pupillenscanner, die Pupillenluminanz, die Krümmung der Pupillenlinse und Linien der Pupillenkapillaren umfasst.

5. Pupillenerkennungsverfahren für eine mobile Vorrichtung gemäß Anspruch 4, wobei bei Verwendung des Pupilleninformations-Erfassungsmoduls (10) zum Erfassen der charakteristischen Pupilleninformation des Benutzers (S21), der auf die zugangsbeschränkte Einheit zugreifen kann, wenn festgestellt wird, dass die erfasste charakteristische Pupilleninformation nicht mit der Anforderung übereinstimmt (S32), der Benutzer aufgefordert wird (S20), die Erfassung erneut durchzuführen (S34), bis mit der Anforderung übereinstimmende charakteristische Pupilleninformation erfasst wird und die mit der Anforderung übereinstimmende charakteristische Pupilleninformation im internen Speicher gespeichert wird.

6. Das Pupillenerkennungsverfahren für eine mobile Vorrichtung gemäß einem der Ansprüche 4 oder 5, wobei es, wenn der Benutzer auf die zugangsbeschränkte Einheit der mobilen Vorrichtung (1) zugreift, folgende Schritte umfasst:
- Auffordern des Benutzers, eine Identitätsprüfung durchzuführen (S20);
- Erfassen von charakteristischer Pupilleninformation des Benutzers durch das Pupilleninformations-Erfassungsmodul (S21);
- Vergleichen der erfassten charakteristischen Pupilleninformation des Benutzers mit der charakteristischen Pupilleninformation, die durch das Master-Kontrollmodul im internen Speicher gespeichert wurde, um zu bestimmen, ob die charakteristische Pupilleninformation diejenige ist, die auf die zugangsbeschränkte Einheit zugreifen darf (S22), und wenn ja, Gewähren des Zugriffs (S23), andernfalls Verweigern des Zugriffs (S24).

7. Pupillenerkennungsverfahren für eine mobile Vorrichtung gemäß einem der Ansprüche 4 bis 6, wobei dieses, wenn das Pupilleninformations-Erfassungsmodul (10) die charakteristische Pupilleninformation des Benutzers erfasst, folgende Schritte umfasst:
- Rücksetzen des Pupilleninformations-Erfassungsmoduls (S30);
- Ansteuern des Pupillenscanners durch den Treiber, um die charakteristische Pupilleninformation des Benutzers zu erfassen (S31);
- Beurteilen, durch den Mikroprozessor, ob das Erfassen der charakteristischen Pupilleninformation beendet ist (S32), und wenn ja, Berechnen der vom Pupillenscanner erfassten charakteristischen Pupilleninformation und Senden derselben an das Master-Kontrollmodul (S33), wenn nicht, dann kontinuierliches Ansteuern des Pupillenscanners, um das Erfassen durchzuführen (S34).

## Revendications

1. Dispositif mobile (1), comprenant :
un module de collecte d'informations de pupille (10) pour collecter des informations caractéristiques de pupille d'un utilisateur ;
un module de contrôle maître (20) pour recevoir les informations caractéristiques de pupille de l'utilisateur quand l'utilisateur accède à une unité contrôlée du dispositif mobile (1), et déterminer, sur la base des informations caractéristiques de pupille de l'utilisateur, si l'utilisateur est autorisé à accéder à l'unité contrôlée ;
et dans lequel
le dispositif mobile (1) est un téléphone mobile ou un assistant numérique personnel ;
l'unité contrôlée comprend des programmes système, des applications ou des fichiers du dispositif mobile (1) ;
le module de collecte d'informations de pupille (10) comprend :
- un lecteur de pupille (100) pour collecter les informations caractéristiques de pupille de l'utilisateur ;
- un dispositif d'entraînement (200) pour entraîner le lecteur de pupille pour collecter les informations caractéristiques de pupille de l'utilisateur ;
- un microprocesseur (300) pour contrôler le lecteur de pupille et le dispositif d'entraînement, calculer les informations caractéristiques de pupille et les envoyer au module de contrôle maître ;
les informations caractéristiques de pupille comprennent la distance entre la pupille et le lecteur de pupille, la luminance de pupille, la courbure du cristallin de pupille, et des lignes des capillaires de pupille.

2. Dispositif mobile (1) selon la revendication 1, dans lequel le lecteur de pupille (100) comprend :
- une unité d'émission/réception d'ultrasons (110) pour obtenir la distance entre la pupille et le lecteur de pupille (100) ;
- une diode photosensible (120) pour collecter la luminance de pupille ;
- une unité d'émission laser (130) pour fournir une source d'éclairage pour faciliter la collecte de la luminance de pupille par la diode photosensible (120) ;
- une unité de traitement du signal (140) pour optimiser les signaux collectés par la diode photosensible (120) ;
- une unité d'interface (150) pour connecter l'unité d'émission/réception d'ultrasons, la diode photosensible (120) et l'unité d'émission laser (130) au microprocesseur (300).

3. Dispositif mobile (1) selon la revendication 2, dans lequel l'unité de traitement du signal (140) comprend au moins un amplificateur (142) et un filtre (144).

4. Procédé de reconnaissance de pupille pour un dispositif mobile (1) selon l'une quelconque des revendications précédentes, comprenant les étapes suivantes :
- paramétrage de la reconnaissance de pupille (S10) pour l'unité contrôlée du dispositif mobile (1) ;
- collecte des informations caractéristiques de pupille (S11) d'un utilisateur pouvant accéder à l'unité contrôlée au moyen du module de collecte d'informations de pupille (10) du dispositif mobile (1) ;
- paramétrage d'une autorisation d'accès (S12) pour les informations caractéristiques de pupille de l'utilisateur au moyen du module de contrôle maître (20) du dispositif mobile (1),
les informations caractéristiques de pupille comprenant la distance entre la pupille et le lecteur de pupille, la luminance de pupille, une courbure du cristallin de pupille, et des lignes des capillaires de pupille.

5. Procédé de reconnaissance de pupille pour un dispositif mobile selon la revendication 4 dans lequel, lors de l'utilisation du module de collecte d'informations de pupille (10) pour collecter les informations caractéristiques de pupille de l'utilisateur (S21) pouvant accéder à l'unité contrôlée, s'il s'avère que les informations caractéristiques de pupille collectées ne correspondent pas à l'exigence (S32), alors l'utilisateur est invité (S20) à effectuer à nouveau une collecte (S34) jusqu'à ce que les informations caractéristiques de pupille correspondant à l'exigence soient collectées, et les informations caractéristiques de pupille correspondant à l'exigence sont stockées dans la mémoire interne.

6. Procédé de reconnaissance de pupille pour un dispositif mobile selon l'une quelconque des revendications 4 et 5 qui comprend, quand l'utilisateur accède à l'unité contrôlée du dispositif mobile (1), les étapes suivantes :
- invitation de l'utilisateur à effectuer une vérification d'identité (S20) ;
- collecte d'informations caractéristiques de pupille de l'utilisateur par le module de collecte d'informations de pupille (S21) ;
- comparaison des informations caractéristiques de pupille collectées de l'utilisateur avec les informations caractéristiques de pupille stockées dans la mémoire interne par le module de contrôle maître pour déterminer si les informations caractéristiques de pupille sont celles qui autorisent l'accès à l'unité contrôlée (S22), si oui, alors autorisation de l'accès (S23), sinon refus de l'accès (S24).

7. Procédé de reconnaissance de pupille pour un dispositif mobile selon l'une quelconque des revendications 4 à 6 qui comprend, quand le module de collecte d'informations de pupille (10) collecte les informations caractéristiques de pupille de l'utilisateur, les étapes suivantes :
- réinitialisation du module de collecte d'informations de pupille (S30) ;
- entraînement du lecteur de pupille par le dispositif d'entraînement pour collecter les informations caractéristiques de pupille de l'utilisateur (S31) ;
- jugement par le microprocesseur que la collecte d'informations caractéristiques de pupille est finie ou non (S32), si oui, calcul des informations caractéristiques de pupille collectées par le lecteur de pupille et envoi de celles-ci au module de contrôle maître (S33), si non, alors entraînement continu du lecteur de pupille pour effectuer une collecte (S34).
